# FASCICULE DE BREVET EUROPEEN

(11) **EP 1 242 043 B1**
(45) Date de publication et mention de la délivrance du brevet: **22.06.2005**
(21) Numéro de dépôt: 00993685.7
(22) Date de dépôt: 19.12.2000
(51) Int. Cl.: A61K 7/28

(54) **COMPOSITION D'HYGIENE BUCCO-DENTAIRE A BASE D'ENZYMES**
ENZYM-ENTHALTENDES ZAHNPFLEGEMITTEL
ENZYME COMPOSITION FOR BUCCO-DENTAL HYGIENE

(30) Priorité: 29.12.1999 FR 9916698
(43) Date de publication de la demande: 25.09.2002
(73) Titulaire: Dana, Jean-Dominique, 06220 Vallauris (FR); Garelli-Milius, Nathalie, 06000 Nice (FR)
(72) Inventeur: Dana, Jean-Dominique, 06220 Vallauris (FR); Garelli-Milius, Nathalie, 06000 Nice (FR)
(74) Mandataire: Hautier, Jean-Louis
(86) Numéro de dépôt international: PCT/FR2000/003591
(87) Numéro de publication internationale: WO 2001/049241

(56) Documents cités:
- EP-A- 0 277 383
- EP-A- 0 451 972
- WO-A-88/02600
- FR-A- 2 651 433
- US-A- 4 150 113

## Description

L'invention a pour objet un procédé de transformation des sucres alimentaires en produits neutres acariogènes ou cariostatiques et la composition pour la mise en oeuvre dudit procédé.

L'invention s'applique à l'hygiène bucco-dentaire.

Cette composition enzymatique peut être utilisée sous forme de dentifrice, gel, pâte, spray, comprimés, tablettes, pastilles, chewing-gum, solution ou toute autre forme usitée en hygiène bucco-dentaire.

La carie dentaire est une maladie infectieuse très répandue dans l'espèce humaine. Elle est due à la présence dans la cavité buccale de micro-organismes bactériens, les streptococcus mutans principalement, qui vont coloniser les surfaces dentaires pour former la plaque dentaire.

L'épaississement de la plaque dentaire par les bactéries est fonction de l'apport alimentaire en sucre (saccharose principalement), car les bactéries possèdent toutes les enzymes de la glycolyse.

Ainsi, l'analyse de la plaque épaissie fait apparaître des polysaccharides issus du métabolisme bactérien du saccharose et qui forment l'essentiel de la matrice.

On distingue :
Polysaccharides extra cellulaires :
   A. Solubles :
      - Dextranes : polymères du glucose formant des chaînes linéaires ∀-1,6 et des branchements multiples ∀-1,3 et parfois ∀-1,2.
      - Levanes : polymères linéaires et parfois ramifiés du fructose. La liaison osidique est de type 3-2,6 et 3-2,4.
      - Glucanes : homopolymères du glucose.
   B. Insolubles :
      - Mutanes : polymères du glucose ∀-1,3 et parfois ∀-1,6
Polysaccharides intrabactériens :
   - Amylopectine :polymères linéaires du glucose formant des chaînes ∀-1,4 et des branchements en ∀-1,6.
La plaque dentaire :
   *La plaque dentaire débute par la formation d'un film organique acellulaire composé d'acides aminés et de glycoprotéines d'origine salivaire et bactérienne. Cette pellicule permanente et non pathogène n'évolue pas si l'alimentation est pauvre en saccharose. Par contre, elle évolue en épaisseur par agglutination et formation de plusieurs couches de glycoprotéines solubles et insolubles à cause d'un régime* *alimentaire riche en sucre.*
   *Les études réalisées en hygiène bucco-dentaire ont permis de mettre en éviden ce la présence de plusieurs souches bactériennes dont le métabolisme glucidique est en grande partie responsable de la formation de la plaque dentaire.*
   *L'alimentation sucrée et la flore bucco-pharyngée constituée principalement par des streptococcus mutans, sanguis et viscosiis, vont hydrolyser les polysaccharides extra cellulaires en fructose et glucose, éléments monomères assemblés par les enzymes bactériennes pour former des chaînes solubles dans un premier temps, et des dextranes insolubles ou mutanes dans un second temps. Des glucanes et levanes contribuent également à la formation de la matrice :*
      *Glucose : dextranes, mutanes et glucanes*
      *Fructose : levanes.*
   *La plaque molle devient mature par accumulation de dextranes visqueuses colonisées par les bactéries (10*^{*8*} *bactéries* / *mg)*.
*Métabolisme de la plaque :*
   *La plaque vit en aérobiose dans un premier temps, pour rapidement être en anaérobiose à l'état mature.*
   *Les levanes sont utilisées comme réserves hydrocarbonées par les bactéries, le saccharose apporté par l'alimentation diffuse à travers la matrice ainsi formée, et fournit le substrat énergétique nécessaire à la survie bactérienne.*
   *Ce métabolisme glucidique de la plaque (glycolyse) aboutit à la formation d'acide lactique dans les conditions anaérobie et provoque une baisse de pH, qui a une incidence directe sur le potentiel cariogène de la plaque dentaire.*
*La Glycclyse :*
   *On désigne par glycolyse, la dégradation des sucres par les bactéries de la plaque dentaire, phénomène déterminant dans la pathogénicité de la carie dentaire.*
   *A partir des sucres, les bactéries synthètisent des polysaccharides extracellulaires (dextranes), du glycogène intracellulaire pour satisfaire leur besoin énergétique, et de l'acide lactique. C'est l'acide lactique qui détruit les cristaux d'hydroxyapatite.*
   *On désigne par antiglycolyse l'inhibition de la formation d'acide lactique à partir de sucres introduits dans la cavité buccale.*
*Les mécanismes de défense :*
   *Les tissus buccaux sont constamment soumis à l'invasion bactérienne provenant de l'alimentation. C'est un environnement chaud et humide qui semble optimal pour assurer la croissance des germes.*
   *Cependant, divers mécanismes de défense sont mis en oeuvre pour préserver l'intégralité des structures.*
   *Les composantes enzymatiques salivaires aident à protéger les structures solides contre les bactéries.*
   *Le système lactoperoxydase, en particulier, produit de l'hypothiocyanate qui a un rôle bactéricide. Son facteur limitant est le peroxyde d'hydrogène qui est souvent fourni de manière très faible à l'état physiologique dans la cavité buccale.*
   Le facteur énergétique est extrêmement important dans la croissance bactérienne. La glycolyse permet la survie et le développement des bactéries.

L'état de la technique peut être défini également par les brevets suivants :
- *FR-2.648.346 : composition dentifrice présentée sous forme solide, à usage humain et animal, caractérisée en ce qu'elle comprend :*
   - *au moins un composant possédant une activité antiseptique et*/*ou anti-microbienne,*
   - *au moins un composant exerçant une action abrasive,*
   - *au moins un composant exerçant une action retard sur le dépôt de tartre,*
   - *au moins un composant possédant la propriété de s'attaquer à la plaque dentaire, et ayant éventuellement des propriétés anti-microbiennes,*
   - *et au moins un composé constituant une source de fluor.*
- *FR-2.651.433 : Complexe enzymatique destiné à être associé à un support, un excipient et*/*ou un véhicule en vue de son application comme produit dentifrice, caractérisé en ce qu'en vue d'exercer simultanément une action d'élimination et*/*ou de retardement sur la formation de la plaque dentaire, du tartre et des caries, il contient au moins une enzyme agissant sur la plaque récente "molle", et au moins une enzyme agissant sur la plaque ancienne insoluble, associées à un groupe d'autres enzymes dont l'enchaînement des actions conduit à la formation d'un milieu fortement bactéricide. Les enzymes dont l'enchaînement d'actions conduit à la formation d'un milieu fortement bactéricide sont l'amyloglucosidase, à raison d'environ 0,8 %, la glucose-oxydase, à raison d'environ 0,2 % et la lactoperoxydase, à raison d'environ 0,02 %.*
- *EP-414.980 : Composition dentifrice liquide, caractérisée en ce qu'elle contient comme ingrédients essentiels : - au moins un composant antiseptique et*/*ou antimicrobien, - au moins un composant agissant sur les impuretés, notamment le tartre, et - au moins un composant constituant une source de fluor, ainsi que les adjuvants usuels, à savoir, de préférence un colorant, un parfum, un astringent et*/*ou un agent de brillance - le tout étant en solution aqueuse, présentée dans un récipient équipé de moyens de projection d'un jet nébulisé, soit par un système mécanique à pompage et refoulement, soit par un gaz propulseur comprimé.*
- *WO 93 10752 : Composition de dentifrice aqueux stabilisée capable de produire ou , en présence de salive, de conduire à* *la production de concentrations antimicrobiennes efficaces d' ions hypothiocyanite. La composition contient à la fois une enzyme oxydoréductase et son substrat spécifique, a fin de produire du peroxyde d'hydrogène d'au moins la concentration efficace minimum. Les compositions de dentifrice aqueux de l'invention peuvent être stabilisées contre l'interaction enzyme*/*substrat prématurée par régulation du niveau d'oxygène dissous dans l'excipient du dentifrice aqueux. On peut facultativement ajouter une enzyme peroxydase afin d'agir sur le peroxyde d'hydrogène précité, oxydant ainsi les ions thiocyanate pour produire les concentrations antimicrobiennes d'ions hypothioxyanate. On peut également ajouter facultativement des ions thiocyanate aux compositions de l'invention en une dose suffisante, avec les autres ingrédients de ladite invention, afin de produire plus d'environ 100 micromoles*/*Iitrelminut-e d'ions hypothiocyanite pendant l'utilisation. La quantité d'eau contenue dans les compositions de dentifrice n'est pas importante pour la stabilité de la composition, à condition de maîtriser le niveau d'oxygène. L'invention concerne également le procédé de production de la composition de dentifrice avec des teneurs minimum en oxygène.*
- *WO 96 40865 : L'invention concerne des souches recombinées de streptococcus mutans, qui se caractérisent par une déficience en production d'acide lactique, ainsi que la préparation d'une déshydrogénase d'alcool recombinée. Ces souches de streptococcus mutans s'utilisent en association avec une méthode de traitement préventif des caries dentaires.*

Le procédé selon l'invention agit comme agent de la prévention de la formation de la plaque dentaire.

Le procédé selon l'invention agit de deux manières : directe et indirecte.
1) L'action principale directe consiste à inhiber la glycolyse en transformant les polyoses cariogènes en polyols non cariogènes (ex. : sorbitol). Cette inhibition intervenant in situ dans la cavité buccale permet d'en stabiliser le pH au-dessus de 6, évitant ainsi la déminéralisation de l'émail dentaire - facteur principal responsable de la carie dentaire.
2) L'action indirecte consiste par l'utilisation des produits issus de la dégradation des polyoses comme substrats par le système enzymatique décrit dans l'invention afin de renforcer la prévention de la formation de la plaque dentaire, par 5 mécanismes :
   2.1 Stimulation du flux salivaire par les polyols,
   2.2 Renforcement du pouvoir anti-bactérien de la salive par activation du système physiologique LPO/Hypothiocyanate par le peroxyde d'hydrogène,
   2.3 Diminution du nombre et de la pathogénicité du milieu bactérien (streptococcus mutans) par création d'un environnement étiologiquement sélectif contre lui par les polyols,
   2.4 Stabilité des δ-lactones, qui ne sont pas des substrats bactériens pour la formation de la plaque dentaire,
   2.5 Reminéralisation de l'émail par la stabilisation de la solution de phosphate de calcium par le sorbitol.

Cette synergie d'actions est à mettre au bénéfice de la nouvelle composition pour l'hygiène bucco-dentaire.

Le procédé selon l'invention consiste à transformer de manière globale la totalité des sucres alimentaires (glucoses, fructoses) par une saccharase (invertase), puis d'agir par une combinaison d'enzymes contenues dans la composition pour l'hygiène bucco-dentaire, sur les D-glucoses et les D-fructoses, ledit complexe enzymatique permet d'obtenir pour :
1- la glucose déshydrogénase ⇒ le D-glucono-*-lactone,
2- la glucose oxydase ⇒ acide D gluconique + H₂O₂, (peroxyde d'hydrogène),
3- la sorbitol déshydrogénase ⇒ le D sorbitol.

Ainsi, le procédé agit simultanément sur les sucres alimentaires, sur la plaque dentaire en transformant les polymères du fructose en sorbitol, sur la plaque dentaire en transformant les polymères du glucose (activation du système de défense physiologique salivaire).
- La composition pour la mise en oeuvre du procédé est essentiellement caractérisée par la combinaison d'un complexe enzymatique suivant :
- une enzyme ou un mélange d'enzymes hydrolysant les liaisons β-2,6 et/ou β-2,1 entre fructofuranosyles (par exemple la saccharase (invertase),
- une oxydo-réductase permettant la transformation du glucose (glucose oxydase et/ou glucodeshydrogénase),
- une oxydo-réductase permettant la transformation du fructose en sorbitol.

Selon un mode préférentiel, l'oxydo-réductase est une enzyme sorbitol déshydrogénase.

Selon un autre mode de réalisation, le complexe enzymatique de la composition est composé des enzymes suivantes :
- invertase,
- glucose-oxydase et/ou glucose déshydrogénase,
- amyloglucosidase,
- sorbitol déshydrogénase.

La composition sous forme liquide, solide (pates, tablettes, chewing-gum, comprimés), de bains de bouche ou nébulosité ou spray pour la mise en oeuvre du procédé est la suivante :

| | |
|---|---|
| Système enzymatique | 1,5% |
| • Invertase | |
| • Glucose oxydase et/ou glucose déshydrogénase | |
| • Amyloglucosidase | |
| • Sorbitol déshydrogénase | |
| Fluor aminé (hydroflurorure de céthylamine) | 0,005% |
| et/ou Fluorure de Na | 0,01% |
| Diméthicone | 1,5% |
| Polysorbate 80 | 1,4% |
| Polysorbate 20 | 1,4% |
| Saccharinate de Na | 0,15% |
| Glycérol | 10% |
| Sorbitol | 40% |
| Xylitol | 5% |
| Silice colloïdale | 1% |
| Parfum | 1% |
| Conservateur | 0,15% |
| Eau | QSP 100% |

La composition sous forme de dentifrice de gel semi-liquide ou sous forme nébulisée est la suivante :

| | |
|---|---|
| Système enzymatique | 1,5% |
| • Invertase | |
| • Glucose oxydase | |
| • Amyloglucosidase | |
| • Sorbitol deshydrogénase | |
| Citrate de zinc | 0,2% |
| Bicarbonate de calcium | 0,1% |
| Xylitol | 15% |
| Sorbitol | 50% |
| Diméthicone | 0,5% |
| Silice colloïdale | 1% |
| Conservateur/antiseptique | 0,5% |
| Saccharinate de Na | 0,2% |
| Arôme de menthe | 1% |
| Eau | QSP 100% |

Les pourcentages et quantités enzymatiques U/g décrits sont donnés à titre indicatif et ne sont pas limitatifs dans les revendications.

De nombreuses études ont montré que le remplacement des sucres rapides (saccharose) par des polyols (sorbitol- xylitol) avait une incidence directe (jusqu'à plus de 80%) sur la diminution des caries. (symposium international - janvier 1988).

### Mécanisme :

La streptoccocus mutans est une bactérie des plus acides, qui ne peut survivre sans saccharose ou glucose disponibles.

Les polyols ne sont pas fermentés en deux acides par les streptoccocus mutans, et entrent en compétition les polyoses pour se constituer à eux dans le métabolisme de la bactérie.

Une des théories, est que le polyol est phosporylé par les streptoccocus mutans lorsqu'il est transporté par la cellule - où il s'accumule - et où le polyol-5-phosphate interrompt le métabolisme normal de la cellule bactérienne et peut altérer son enveloppe extérieure.

Les bactéries survivent dans la cavité buccale, parce qu'elles peuvent tolérer des modifications dans les conditions de leur environnement.

Les polyols rendent les bactéries plus sensibles à leur environnement et moins aptes à devenir dominantes dans la plaque dentaire ou les sites carieux. On note une réduction de la plaque dans les expérimentations où les polyols remplacent ou sont en compétition avec les polyoses, ainsi qu'une réduction significative du nombre de streptoccocus mutans.

D'autre part, les polyols stimulent la sécrétion salivaire, diminuent le taux d'acidité, participent avec le Ca++, par formation de complexes, à des effets directs sur la reminéralisation de l'émail.

En résumé, les polyols sont des sucres non cariogènes possédant un potentiel de propriétés cariostatiques.

Le remplacement ou la transformation dans la bouche de saccharose en polyols (sorbitol) apparaît pour affecter plus d'un processus normalement nécessaire dans le développement des lésions cariogènes.

Jusqu'à ce jour, seule la substitution du sucre par des polyols existait (chewing-gum, diététique).

Grâce au processus de l'invention décrite ci-après, la transformation du sucre cariogène apporté dans l'alimentation en polyols non cariogènes, se fera spécifiquement au niveau de la plaque dentaire et dans la cavité buccale, pour diminuer de manière significative la glycolyse.

### Les antibactériens

Les antibactériens (chlorexidine, hexomedine, trichlosan, triclarban...) ont été les premiers produits utilisés dans la lutte contre la plaque dentaire et la carie, comme agents antiseptiques pour combattre la prolifération bactérienne.

Ils présentent un inconvénient majeur : leur utilisation systématique entraîne des mutations bactériennes avec apparition de microbes poly-résistants.

### Les enzymes

Différents types d'enzymes ont été proposés et peuvent être regroupés en trois grandes catégories.

### Système enzymatique anti-bactérien

Plusieurs brevets ont décrit des systèmes antibactériens par renforcement du système lactopéroxydase salivaire.

Le brevet Laclède N° EP 133736 supplée ainsi la salive en apportant de la lactopéroxidase exogène, de la glucose oxydase et du thiocyanate de potassium.

L'inconvénient majeur réside dans la nécessité de rajouter du glucose dans la composition qui est un substrat du métabolisme bactérien.

Système d'enzymes antiplaque agissant directement sur la plaque dentaire :

Plusieurs brevets décrivent des systèmes enzymatiques qui interviennent directement sur les polymères constitutifs de la plaque dentaire soit les dextranes, les mutanes et les glucanes.

Le brevet FR-A-2502958 décrit une combinaison mutanase et dextranase qui agit par hydrolyse spécifique des liaisons polyglucosidiques des mutanes (insolubles) et des dextranes (solubles).

Le brevet FR-A-89 11868 propose également l'hydrolyse enzymatique des mutanes et dextranes, mais renforce et étend cette action par utilisation conjointe d'amyloglucosidase qui agit spécifiquement sur l'amylopectine.

Il utilise le glucose obtenu par ces différentes hydrolyses, pour former du peroxyde d'hydrogène utilisé par la lactopéroxydase du complexe exogène, et dégager de l'oxygène radicalaire capable d'oxyder des thiocyanates et hypothiocyanates (système LP), qui sont de puissants bactéricides susceptibles d'intervenir sur les streptoccocus mutans.

En ce qui concerne les polymères de fructose de la plaque dentaire, rien n'est décrit dans l'art antérieur.

### Systèmes d'enzymes antiglycolyse

Peu de brevets n'interviennent dans le blocage complet de la glycolyse bactérienne :
Certains interviennent de manière indirecte en ayant une action antibactérienne.
D'autres interviennent en luttant contre les conséquences de cette glycolyse sur la modification de Ph.

Aucun système enzymatique actuel n'agit de manière globale et ne transforme la totalité des dérivés dés sucres cariogènes en substances neutres ou acariogènes.

C'est le but de la présente invention.

### Description de l'invention :

La présente invention concerne un groupe d'enzymes devant être utilisés pour inhiber la glycolyse au niveau bucco-dentaire, et comme agents de prévention directe ou indirecte de la formation de la plaque dentaire.

Jusqu'à présent tous les enzymes utilisés dans ce domaine, interviennent uniquement sur le glucose ou les polymères de glucose (mutanes, dextranes, amylopectine...) qu'ils hydrolysent pour générer des unités glucose.

Seul le brevet FR-A- 89 11868 transforme ces unités glucose pour former de l'hypothiocyanate bactéricide grâce au système LP.

Le complexe enzymatique de la présente invention permet d'inhiber la glycolyse en transformant les sucres cariogènes en éléments acariogènes ou neutres, et indirectement de renforcer la prévention de la formation de la plaque dentaire.

### Cette nouvelle composition enzymatique agit sur :

Le saccharose alimentaire pour donner du glucose et du fructose
1. Les levanes de la plaque pour donner des unités fructose
2. Les unités fructose obtenues soit par dégradation des levanes, soit par hydrolyse du saccharose pour donner des produits acariogènes
3. Les unités glucose obtenus soit par dégradation de la plaque dentaire (dextranes, mutanes, glucanes, amylopectine), soit par hydrolyse du saccharose pour donner des produits inertes.

La transformation du saccharose alimentaire en unités fructose et glucose est réalisée par une invertase telle que la saccharase par exemple.

La dégradation des polymères de fructose des levanes en unités fructose peut être réalisée par un mélange d'enzymes hydrolysant les liaisons β-2,6 et/ou β-2,1 entre fructofuranosyles.

Les unités fructose obtenues par inversion du saccharose alimentaire, et/ou hydrolyse des levanes sont utilisées comme substrat par une oxydo-réductase telle que le sorbitol déhydrogénase (EC 1.1.1.14) par exemple - qui réduit ces unités fructose en polyol, à savoir le sorbitol. Dans ce cas, la dégradation enzymatique de la plaque dentaire (levanes en particulier) permet d'obtenir du sorbitol dont la propriété acariogène est connue de l'homme de l'art.

Les unités glucose obtenues, soit par inversion du saccharose alimentaire, soit par hydrolyse enzymatique des polyglucosides constitutifs de la plaque dentaire, peuvent être utilisées comme substrat par une oxydo-réductase-glucose déhydrogénase par exemple - pour être réduites en D-glucono-*-lacone neutre et/ou utilisés comme substrats par une glucose-oxydase pour donner de l'acide glucuronique et du peroxyde d'hydrogène. Ce dernier devient le facteur facilitant du système lactopéroxydase salivaire, et contribue à l'obtention d'hypothiocyanate antibactérien physiologique.

## Revendications

1. Composition pour améliorer l'hygiène bucco-dentaire notamment en nettoyant la plaque dentaire **caractérisée par le fait**
**qu'**elle est essentiellement composée par la combinaison d'un complexe enzymatique suivant :
- une enzyme ou un mélange d'enzymes hydrolysant les liaisons β-2,6 et/ou β-2,1 entre fructofuranosyles,
- une oxydo-réductase permettant la transformation du glucose (glucose oxydase et/ou glucodeshydrogénase),
- une oxydo-réductase permettant la transformation du fructose en sorbitol.

2. Composition selon la revendication 1, **caractérisée par le fait**
**que** le complexe enzymatique de la composition est composé des enzymes suivantes :
- invertase,
- glucose-oxydase et/ou glucose déshydrogénase,
- amyloglucosidase,
- sorbitol déshydrogénase.

3. Composition selon la revendication 2 **caractérisée par le fait**
**que** l'oxydo-réductase permettant la transformation du fructose est une enzyme sorbitol déshydrogénase.

4. Composition selon l'une quelconque des revendications 1 ou 2 **caractérisée par le fait**
**que** la composition sous forme liquide, de bains de bouche ou nébulosité ou spray pour la mise en oeuvre du procédé est la suivante :
| | |
|---|---|
| Système enzymatique | 1,5% |
| • Invertase | |
| • Glucose oxydase et/ou glucose déshydrogénase | |
| • Amyloglucosidase | |
| • Sorbitol déshydrogénase | |
| Fluor aminé (hydroflurorure de céthylamine) et/ou Fluorure de Na 0,01% | 0,005% |
| Diméthicone | 1,5% |
| Polysorbate 80 | 1,4% |
| Polysorbate 20 | 1,4% |
| Saccharinate de Na | 0,15% |
| Glycérol | 10% |
| Sorbitol | 40% |
| Xylitol | 5% |
| Silice colloïdale | 1% |
| Parfum | 1% |
| Conservateur | 0,15% |
| Eau | QSP 100% |

5. Composition selon l'une quelconque des revendications 1 ou 2 **caractérisée par le fait**
**que** la composition sous forme de dentifrice de gel semi-liquide ou sous forme nébulisée est la suivante :
| | |
|---|---|
| Système enzymatique | 1,5% |
| • Invertase | |
| • Glucose oxydase | |
| • Amyloglucosidase | |
| • Sorbitol deshydrogénase | |
| Citrate de zinc | 0,2% |
| Bicarbonate de calcium | 0,1% |
| Xylitol | 15% |
| Sorbitol | 50% |
| Diméthicone | 0,5% |
| Silice colloïdale | 1% |
| Conservateur/antiseptique | 0,5% |
| Saccharinate de Na | 0,2% |
| Arôme de menthe | 1% |
| Eau | QSP 100% |

## Patentansprüche

1. Zusammensetzung zur Verbesserung der Mund- und Zahnhygiene,
insbesondere durch Reinigung der Plaque, **gekennzeichnet dadurch, dass** sie im wesentlichen durch Kombination des folgenden Enzymkomplexes hergestellt wird:
- ein Enzym oder eine Enzymmischung, welche die β-2,6- und/oder β-2,1-Verbindungen zwischen Fructofuranosylgruppen hydrolysiert,
- eine Oxidoreduktase, welche die Umwandlung der Glucose ermöglicht (Glucoseoxidase und/oder Glucosedehydrogenase),
- eine Oxidoreduktase zur Umwandlung der Fructose in Sorbitol.

2. Zusammensetzung gemäß Anspruch 1, **gekennzeichnet dadurch,**
**dass** der Enzymkomplex der Zusammensetzung aus folgenden Enzymen besteht:
- Invertase
- Glucoseoxidase und/oder Glucosedehydrogenase
- Amyloglycosidase,
- Sorbitol Dehydrogenase.

3. Zusammensetzung gemäß Anspruch 2, **gekennzeichnet dadurch,**
**dass** das Enzym Sorbitol-Dehydrogenase die zur Umwandlung der Fructose dienende Oxidoreduktase ist.

4. Zusammensetzung gemäß einem der Ansprüche 1 oder 2, **gekennzeichnet dadurch, dass** die zur Anwendung des Verfahrens in flüssiger Form, als Mundspülung, in nebulöser Form oder als Spray dienende Zusammensetzung, die folgende ist:
| | |
|---|---|
| Enzymsystem | 1,5 % |
| - Invertase | |
| - Glucoseoxidase und/oder Glucosedeydrogenase | |
| - Amyloglucosidase | |
| - Sorbitol Dehydrogenase | |
| Aminfluor (Cetylamin-hydrofluorid) | 0,005 % |
| und/oder Natriumfluorid | 0,01 % |
| Dimeticon | 1,5 % |
| Polysorbat 80 | 1,4 % |
| Polysorbat 20 | 1,4 % |
| Natriumsaccharinat | 0,15 % |
| Glycerol | 10 % |
| Sorbitol | 40 % |
| Xylitol | 5 % |
| Kolloïdales Siliziumdioxid | 1 % |
| Parfum | 1 % |
| Konservierungsstoff | 0,15 % |
| Wasser | q.s. 100 % |

5. Zusammensetzung gemäß einem der Ansprüche 1 oder 2, **gekennzeichnet dadurch,**
**dass** die in Form von Zahncreme, halbflüssigem Gel oder nebulöser Form vorliegende Zusammensetzung die folgende ist:
| | |
|---|---|
| Enzymsystem | 1,5% |
| - Invertase | |
| - Glucoseoxidase | |
| - Amyloglucosidase | |
| - Sorbitol Dehydrogenase | |
| Zinkcitrat | 0,2 % |
| Calciumbicarbonat | 0,1 % |
| Xylitol | 15 % |
| Sorbitol | 50 % |
| Dimeticon | 0,5 % |
| Kolloidales Siliziumdioxid | 1 % |
| Konservierungsstoff / Antiseptikum | 0,5 % |
| Natriumsaccharinat | 0,2 % |
| Pfefferminzaroma | 1 % |
| Wasser | q.s. 100 % |

## Claims

1. Composition to improve dental hygiene in particular by cleaning bacterial plaque **characterized by** the fact
that it is essentially composed of the combination of an enzymatic compound as follows:
- an enzyme or a mixture of enzymes hydrolyzing the liaisons β-2.6 and/or β-2.1 between fructofuranosyles,
- an oxidoreductase allowing the transformation of glucose (glucose oxidase and/or glucodehydrogenase),
- a oxidoreductase allowing the transformation of fructose into sorbitol.

2. Composition according to claim 1, **characterized by** the fact
that the enzymatic complex of the composition is composed of the following enzymes:
- invertase,
- glucose-oxidase and/or glucose dehydrogenase,
- amyloglucosidase,
- sorbitol dehydrogenase.

3. Composition according to claim 2, **characterized in that**
the oxydoreductase allowing the transformation of the fructose is a sorbitol dehydrogenase enzyme.

4. Composition according to any of claims 1 or 2 **characterized in that**
**that** the composition in liquid, mouth wash or milk or spray form for the application of the process is as follows:
| | |
|---|---|
| Enzymic system | 1.5% |
| - invertase | |
| - Glucose oxidase and/or glucose dehydrogenase | |
| - Amyloglucosidase | |
| - Sorbitol dehydrogenase | |
| Amine fluoride (cethylamine hydrofluoride) | 0.005% |
| And/or sodium fluoride | 0.01 % |
| Dimethicone | 1.5% |
| Polysorbate 80 | 1.4% |
| Polysorbate 20 | 1.4% |
| Sodium saccharinate | 0.15% |
| Glycerol | 10% |
| Sorbitol | 40% |
| Xylitol | 5% |
| Silica colloidal | 1% |
| Aroma | 1% |
| Preservative | 0.15% |
| Water | QS 100% |

5. Composition according to any of claims 1 or 2 **characterized in that** the composition in the form of semi-liquid toothpaste or milk is as follows:
| | |
|---|---|
| Enzymic system | 1.5% |
| - Invertase | |
| - Glucose oxidase | |
| - Amyloglucosidase | |
| - Sorbitol dehydrogenase | |
| Zinc citrate | 0.2% |
| Calcium bicarbonate | 0.1 % |
| Xylitol | 15.0% |
| Sorbitol | 50% |
| Dimethicone | 0.5% |
| Silica colloidal | 1% |
| Preservative/antiseptic | 0.5% |
| Sodium saccharinate | 0.2% |
| Mint aroma | 1% |
| Water | QS 100% |
